# EUROPÄISCHE PATENTANMELDUNG

(11) **EP 1 103 251 A1**
(43) Veröffentlichungstag der Anmeldung: **30.05.2001**
(21) Anmeldenummer: 00124974.7
(22) Anmeldetag: 16.11.2000
(51) Int. Cl.: A61K 7/48

(54) **Verwendung von 1,1-Dioxo-perhydro-1,2,4-thiadiazinen**

(30) Priorität: 29.11.1999 DE 19957383
(71) Anmelder: Goldschmidt AG, 45127 Essen (DE)
(72) Erfinder: Dietz, Thomas, Dr., 45259 Essen (DE); Grüning, Burghard, Dr., 45134 Essen (DE); Lersch, Peter, Dr., 46537 Dinslaken (DE); Weitemeyer, Christian, Dr., 45134 Essen (DE)

(57) **Zusammenfassung**

Verwendung von 1,1-Dioxo-perhydro-1,2,4-thiadiazinen der allgemeinen Formel (I), 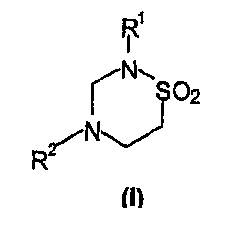 worin R¹ und R², die gleich oder verschieden sind, Wasserstoffatome, Alkylreste mit 1 bis 18 Kohlenstoffatomen, Cycloalkylreste, Aralkylreste oder aromatische oder heterocyclische Reste jeweils mit 3 bis 20 C-Atomen bedeuten, wobei die Reste R¹ und R² gegebenenfalls substituiert sind, zur Herstellung kosmetischer oder pharmazeutischer Zubereitungen für die Behandlung von unreiner Haut und Akne.

## Beschreibung

Die Erfindung betrifft die Verwendung von 1,1-Dioxoperhydro-1,2,4-thiadiazinen zur Herstellung von kosmetischen oder pharmazeutischen Zubereitungen, die gegen unreine Haut und Formen der Akne wirksam sind.

Unreine Haut wird im wesentlichen durch eine erhöhte Aktivität der Talgdrüsen und einer damit verbundenen erhöhten Absonderung von Sebum verursacht. Akne ist eine Hauterkrankung, die durch nicht-entzündliche und entzündliche Knötchen gekennzeichnet ist, die zur Pustel-, Abszess- und Narbenbildung führen kann. Ursachen sind die Verhornung und Verstopfung der Haarfollikel (Bildung von Komedonen), eine erhöhte Talgproduktion und die Produktion gewebeschädigender Enzyme durch Bakterien (Propionibacterium acnes). Therapeutisch kommen Antibiotika, Keratolytika und Peroxide zur Anwendung.

So beschreibt beispielsweise die EP 0 536 360 A die topische Behandlung von Akne mit den Antibiotikaklassen Aminopenicilline und Cephalosporine. Antibiotika haben jedoch den Nachteil, dass die bekämpften Mikroorganismen, beispielsweise Bakterien, dagegen resistent werden können. Die EP 0 639 068 A beschreibt die Behandlung von Akne mit den Keratolytika Salicylsäure und Pantothensäure. Keratolytika erweichen oder lösen verhornte Hautschichten auf, haben jedoch keinen Einfluss auf Entzündungen verursachende Mikroorganismen. US 5 767 098 A beschreibt die Behandlung von Akne durch topische Anwendung einer Kombination eines Antibiotikums mit einem Peroxid. Substanzen wie Benzoylperoxid sind jedoch Radikalbildner, die oxidativen Stress der Haut verursachen und damit zu einer Langzeitschädigung führen können. Substanzen wie Benzoylperoxid oder Retinsäure erweisen sich zwar als wirksam bei der Reduktion von Pickeln, verursachen aber auch Reizungen, Sensibilisierungen und Allergien, die sich als Stechen, Jucken, Brennen, Abschuppen oder Rötungen der Haut äußern.

Taurolidin ist ein 1,1-Dioxo-perhydro-1,2,4-thiadiazin-Derivat und eine seit über 30 Jahren bekannte Substanz (siehe CH 482 713 A)und durch die allgemeine Formel (I) 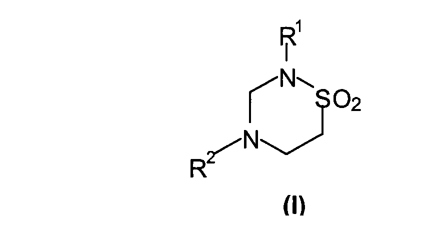 charakterisiert, wobei im Fall von Taurolidin (I') R¹ = H und R² ein Rest der allgemeinen Formel (II) 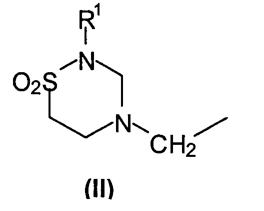 ist. Sie fand bisher ausschließlich Anwendung in der Humanmedizin. So beschreibt beispielsweise EP 0 253 662 A die Verwendung von Taurolidin in Form einer wässrigen Lösung zur parenteralen Verabreichung bei chirurgischen Eingriffen gegen Infektionen durch Bakterien oder bakterielle Toxine. WO 92/00743 beschreibt ein Verfahren zur Behandlung oder Prophylaxe von Tumoren durch Verabreichung einer effektiven Dosis von Taurolidin und/oder Taurultam. In der DE 35 33 612 A wird die Verwendung von Taurolidin als Blutgerinnung hemmendes Mittel beschrieben. WO 94/03174 beschreibt ein Taurolidin enthaltendes zahnmedizinisches Mittel zur Behandlung von beispielsweise Parodontitis.

Die Wirkungsweise von Taurolidin beruht vermutlich auf der Übertragung von Methylolgruppen auf die Hydroxyl- oder Aminogruppen, die sich auf Toxinen oder auf dem Murein von Bakterienzellwänden befinden. In Lösung liegt Taurolidin (I') mit R¹ = H und R² = Rest mit der Formel (II) im Gleichgewicht mit Taurultam (I") mit R¹ und R² = H und N-Methyloltaurultam (I''')mit R¹ = H und R² = -CH₂-OH vor. Bei der Übertragung der Methylolgruppen auf Toxine oder Bakterien wird Methyloltaurultam (I''') in Taurultam (I") umgewandelt, das wiederum sich im Gleichgewicht mit Methyloltaurinamid (III)

R³HN―CH₂―CH₂―SO₂―R⁴ (III)

mit R³ = -CH₂OH und R⁴ = -NH₂ befindet. Durch Methylolgruppenübertragung wird Methyloltaurinamid in Taurinamid (III') mit R³ = H und R⁴ = -NH₂ umgewandelt. Taurinamid (III') wird physiologisch in Taurin (III") mit R³ = H und R⁴ = OH transformiert, eine natürlich vorkommende Aminosulphonsäure, die ausgesprochen gut vom Körper toleriert wird.

Aus der Literatur ist bekannt, dass 1,1-Dioxo-perhydro- 1,2,4-thiadiazine, insbesondere Taurolidin und die oben genannten Metaboliten, wirksam gegen verschiedene Bakterien und Pilze sind. So wird in Arzneim.-Forschung, 42 (II), 1992, 1157 - 1159 die In-vitro-Aktivität von Taurolidin gegen oralpathogene Mikroorganismen beschrieben. In der Untersuchung ist auch das anaerobe Propionibacterium acnes berücksichtigt, das man außer in entzündeten Stellen im Zahnfleisch auch in Entzündungsherden bei Akne-Patienten findet. Allerdings stellen das Auftreten von Propionibacterium acnes bei Akne-Patienten und die dadurch verursachten Entzündungen eine Folgeerscheinung und keine Ursache von Akne dar. Der Literatur lässt sich nichts über die Verwendung von Taurolidin zur topischen Anwendung auf der Haut zum Zwecke der Behandlung unreiner Haut und Akne entnehmen.

Aufgabe der vorliegenden Erfindung ist es daher, einen Wirkstoff bereitzustellen, der den Ursachen unreiner Haut und Akne, nämlich Hyperkeratosis, übermäßige Sebumproduktion und bakterielle Infektion, entgegenwirkt, jedoch keine negativen Auswirkungen auf die Haut wie beispielsweise Hautirritationen, Hautschuppung oder Resistenz von Bakterien besitzt.

Überraschenderweise wurde nun gefunden, dass 1,1-Dioxoperhydro-1,2,4-thiadiazine der allgemeinen Formel (I), 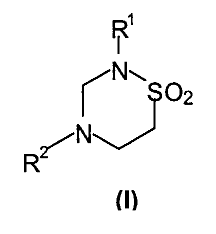 insbesondere Taurolidin (I') mit R¹ = H und R² = Rest mit der Formel (II) 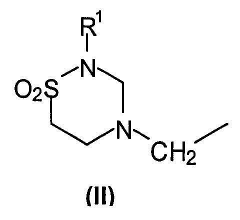 oder Taurultam (I") mit R¹ und R² = H gegen unreine Haut und Formen der Akne außerordentlich wirksam sind, jedoch als Chemotherapeutika nicht die Nachteile von Antibiotika, beispielsweise die Ausbildung von Resistenzen, besitzen, und zusätzlich bei topischer Applikation auf der Haut keinerlei Reizwirkungen auslösen. Die gefundene hohe Wirksamkeit gegen unreine Haut und Akne lässt sich nicht allein mit der in vitro Aktivität von Taurolidin gegen Propionibacterium acnes erklären, und war deshalb nicht zu erwarten.

Gegenstand der Erfindung ist in einer ersten Ausführungsform die Verwendung von 1,1-Dioxo-perhydro-1,2,4-thiadiazinen der allgemeinen Formel (I), 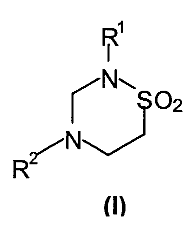 worin R¹ und R², die gleich oder verschieden sind, Wasserstoffatome, Alkylreste mit 1 bis 18 Kohlenstoffatomen, Cycloalkylreste, Aralkylreste oder aromatische oder heterocyclische Reste jeweils mit 3 bis 20 C-Atomen bedeuten, wobei die Reste R¹ und R² gegebenenfalls substituiert sind, zur Herstellung kosmetischer oder pharmazeutischer Zubereitungen für die Behandlung von unreiner Haut und Akne. Entsprechende Verbindungen, insbesondere Verbindungen mit substituierten Resten sind aus der CH 48 27 13 A bekannt, auf die voll inhaltlich Bezug genommen wird.

Ein weiterer Gegenstand der Erfindung ist die Verwendung von 1,1-Dioxo-perhydro-1,2,4-thiadiazinen (I) in kosmetischen oder pharmazeutischen Zubereitungen zur Behandlung von unreiner Haut und Akne, dadurch gekennzeichnet, dass in dem 1,1-Dioxoperhydro-1,2,4-thiadiazin (I) R¹ und R² Wasserstoffatome sind (Taurultam I") oder R¹ ein Wasserstoffatom ist und R² einem Rest der Formel (II) entspricht (Taurolidin I').

Die Konzentration an 1,1-Dioxo-perhydro-1,2,4-thiadiazin (I)einschließlich Metaboliten in den kosmetischen oder pharmazeutischen Zubereitungen beträgt vorzugsweise 0,005 bis 10,00 Gew.-%, bevorzugt 0,01 - 5,0 Gew.-%, jeweils bezogen auf das Gesamtgewicht der Zubereitungen.

Nach einer vorteilhaften Ausführungsform der vorliegenden Erfindung werden 1,1-Dioxo-perhydro-1,2,4-thiadiazine (I) mit anderen gegen unreine Haut und Akne wirksamen Mitteln in kosmetischen oder pharmazeutischen Zubereitungen eingesetzt. Besonders geeignet ist eine Kombination von 1,1-Dioxo-perhydro- 1,2,4-thiadiazin (I) mit einem Keratolytikum, insbesondere Salicylsäure, Milchsäure, Glycolsäure, Citronensäure oder Äpfelsäure.

Die Zubereitungen können in Form von flüssigen Zusammensetzungen angewendet werden, die mittels Pinseln, Abstreifern oder Roll-on-Vorrichtungen auftragbar sind, als Stifte und in Form von W/O- oder O/W-Emulsionen, die aus üblichen Flaschen und Behältern auftragbar sind, beispielsweise Cremes oder Lotionen. Weiterhin können die Zubereitungen vorteilhaft in Form von Gesichtswässern, Tinkturen oder Reinigungsformulierungen vorliegen.

Als übliche Trägerstoffe zur Herstellung der Zubereitungen können neben Wasser, Ethanol und Isopropanol, Glycerin, Propylenglycol, Polyglycerin und Polyethylenglycol, hautpflegende Fette oder fettähnliche Stoffe wie Cetylalkohol, Cetearyloctanoat, Decyloleat und Octyldodecanol, in den für solche Präparate üblichen Mengenverhältnissen eingesetzt werden, sowie Verdickungsmittel, beispielsweise Hydroxyethyl- oder Hydroxypropylcellulose, Salze der Polyacrylsäure, Polyvinylpyrrolidon, daneben aber auch in kleinen Mengen cyclische und kettenförmige Siliconöle sowie organisch modifizierte Silicone.

Als Emulgatoren zur Herstellung der Zubereitungen, welche vorteilhaft als flüssige Zubereitungen auf die gewünschten Hautbereiche aufgetragen werden sollen, und die in den Zubereitungen in geringer Menge, beispielsweise 1 bis 5 Gew.-%, bezogen auf die Gesamt-Zusammensetzung, verwendet werden können, haben sich nichtionogene Typen wie Polyoxyethylen-Fettalkoholether, beispielsweise Cetostearylalkoholpolyethylenglycolether mit 10, 20 oder 25 angelagerten Ethylenoxid-Einheiten pro Molekül, Cetostearylalkohol sowie Sorbitanester und Sorbitanester-Ethylenoxid-Verbindungen (beispielsweise Sorbitan-monostearat und Polyoxyethylensorbitanmonostearat), Glycerin- und Polyglycerinester, beispielsweise Polyglycerin-Isostearat, Zuckerester, beispielsweise Methylglucosedioleat oder Cetearylglucosid, Polysiloxan-Polyoxyethylen/oxypropylen- und Polysiloxan-Polyoxyethylen/oxypropylen-Polycetyl-Copolymere als geeignet erwiesen.

Zusätzlich zu den genannten Bestandteilen können den Zubereitungen gemäß der Erfindung, deren pH-Wert vorzugsweise beispielsweise durch übliche Puffergemische auf 3,5 bis 9,0 insbesondere 4,0 bis 6,5 eingestellt wird, Parfüm, Farbstoffe, Antioxidantien (beispielsweise α-Tocopherol und seine Derivate oder Butylhydroxytoluol (BHT), 2,6-Di-tert.-butyl-4- methylphenol) in Mengen von 0,01 bis 0,03 %, bezogen auf die Gesamtzusammensetzung), Dispergierhilfsmittel, Puffergemische oder andere übliche kosmetische Rohstoffe beigemischt werden.

Die jeweils einzusetzenden Mengen an Trägerstoffen können in Abhängigkeit von der Art des jeweiligen Produktes vom Fachmann durch einfaches Ausprobieren leicht ermittelt werden.

Die Herstellung der erfindungsgemäßen Zubereitungen erfolgt abgesehen von speziellen Zubereitungen, die in den Beispielen jeweils gesondert vermerkt sind, in üblicher Weise, zumeist durch einfaches Vermischen unter Rühren, gegebenenfalls unter leichter Erwärmung. Für Emulsionen werden Fettphase und die Wasserphase beispielsweise separat, gegebenenfalls unter Erwärmen hergestellt und dann emulgiert. Ansonsten sind die üblichen Maßregeln für das Zusammenstellen von galenischen Formulierungen zu beachten, die dem Fachmann geläufig sind.

Es folgen vorteilhafte Ausführungsbeispiele der vorliegenden Erfindung.

### Beispiele:

### Beispiel 1:

| Gel | |
|---|---|
| | Gew.-% |
| Taurolidin | 2,0 |
| Hydroxyethylcellulose | 2,0 |
| Wasser | 96,0 |

Taurolidin wurde in Wasser gelöst und die Hydroxyethylcellulose eingearbeitet. Das entstandene Gel wurde in Aluminiumtuben abgefüllt.

### Beispiel 2:

| Wachsstift | |
|---|---|
| | Gew.-% |
| Hydriertes Rizinusöl | 5,0 |
| Bienenwachs | 6,0 |
| Hartparaffin | 30,0 |
| C₁₂ bis C₁₅ Alkylbenzoat | 17,0 |
| Taurolidin | 0,9 |
| Octyldodecanol | 41,1 |

Die Bestandteile wurden bei etwa 75 °C aufgeschmolzen, gut vermischt und in geeignete Formen gegossen.

### Beispiel 3:

| Gesichtswasser | |
|---|---|
| | Gew.-% |
| Ethanol | 8,0 |
| Taurolidin | 2,0 |
| Wasser | 90,0 |

### Beispiel 4:

| O/W-Gesichtscreme | | |
|---|---|---|
| | | Gew.-% |
| A | Polyglyceryl-3-methylglucose-distearat | 3,0 |
| | Glyceryl-Stearat | 2,8 |
| | Stearyl-Alkohol | 1,2 |
| | Isocetyl-Palmitat | 6,0 |
| | Ethylhexyl-Palmitat | 6,0 |
| | Isopropyl-Palmitat | 6,0 |
| | | |
| B | Glycerin | 3,0 |
| | Wasser | 70,0 |
| | Taurolidin | 2,0 |

Phase A und B wurden separat auf 70 °C erwärmt. Phase A wurde unter Rühren zu Phase B gegeben und anschließend homogenisiert. Unter leichtem Rühren wurde abgekühlt.

### Beispiel 5:

| W/O-Creme | | |
|---|---|---|
| | | Gew.-% |
| A | Cetyl-Dimethicon-Copolyol | 2,0 |
| | Polyglyceryl-4-isostearat | 1,0 |
| | Mineralöl | 12,0 |
| | Ethylhexylstearat | 5,0 |
| | Hydriertes Rizinusöl | 0,8 |
| | mikrokristallines Wachs | 1,2 |
| | | |
| B | Natriumchlorid | 1,0 |
| | Taurolidin | 1,5 |
| | Wasser | 75,5 |

Phase A wurde auf ca. 80 °C erwärmt, Phase B langsam eingerührt und dann kurz homogenisiert. Unter leichtem Rühren wurde auf unter 30 °C abgekühlt und erneut homogenisiert.

### Beispiel 6:

| W/O/W-Emulsion | | |
|---|---|---|
| | | Gew.-% |
| O | Cetyl-Dimethicon-Copolyol | 2,0 |
| | Capryl/Caprin-Triglyceride | 16,0 |
| | Salicylsäure | 2,0 |
| | | |
| W1 | Glycerin | 1,0 |
| | Magnesiumsulfat-Heptahydrat | 0,15 |
| | Taurolidin | 1,0 |
| | Wasser | 27,85 |
| | | |
| W2 | Wasser | 39,0 |
| | Propylenglycol | 2,0 |
| | Caprylyl/Capryl-Glucoside, 10 % | 8,0 |
| | Natriumhydroxid, 10 % | q.s. |
| | | |
| D | Xanthan-Gum | 0,1 |
| | Acrylat/C10-30 Alkyl-Acrylat-Crosspolymer | 0,2 |
| | Mineralöl | 0,7 |

Phase W1 wurde unter Rühren zu Phase O gegeben und anschließend homogenisiert. Die Komponenten von Phase W2 wurden gemischt. Aus den Komponenten von Phase D wurde eine Dispersion hergestellt, zu W2 gegeben und kurz homogenisiert. Die W1/O-Emulsion wurde portionsweise unter Rühren zu W2 geben.

Die überraschend hohe Wirksamkeit von 1,1-Dioxo-perhydro-1,2,4- thiadiazinen, insbesondere Taurolidin, in der Behandlung unreiner Haut und Akne wird beispielhaft durch folgende Fallbeschreibungen belegt.

### Fall 1:

Fall 1 ist ein 18-jähriges Mädchen mit Akne simplex. Seit zwei Jahren wurde sie mit gängigen Substanzen (Benzoylperoxid, Aluminiumoxid als Schältherapie) behandelt, allerdings ohne Erfolg. Sie wendete zweimal pro Tag das Gel aus Beispiel 1 über einen Zeitraum von 3 Monaten an. Schon nach zwei Wochen zeigte sich ein positiver Verlauf, sichtbar am Rückgang der entzündeten Stellen und der infizierten Pusteln. Nach 3 Monaten hatte sich der Zustand fundamental verbessert. Es war keine Behandlung mehr mit dem Taurolidin-Gel erforderlich. Hautirritationen wurden während der gesamten Behandlungsdauer nicht beobachtet.

### Fall 2:

Fall 2 ist ein 34-jähriger Mann mit starker Akne und Komedonen am Rücken. Er wurde 8 Tage mit dem Gel aus Beispiel 1 behandelt. Schon nach einem Tag konnte ein Rückgang der infizierten Stellen beobachtet werden. Nach drei Tagen war bereits eine wesentliche Besserung der Beschwerden eingetreten. Nach einer Woche war keine Behandlung mehr erforderlich.

## Patentansprüche

1. Verwendung von 1,1-Dioxo-perhydro-1,2,4-thiadiazinen der allgemeinen Formel (I), 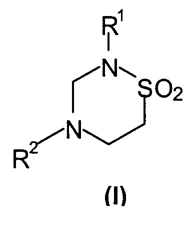 worin R¹ und R², die gleich oder verschieden sind, Wasserstoffatome, Alkylreste mit 1 bis 18 Kohlenstoffatomen, Cycloalkylreste, Aralkylreste oder aromatische oder heterocyclische Reste jeweils mit 3 bis 20 C-Atomen bedeuten, wobei die Reste R¹ und R² gegebenenfalls substituiert sind, zur Herstellung kosmetischer oder pharmazeutischer Zubereitungen für die Behandlung von unreiner Haut und Akne.

2. Verwendung nach Anspruch 1, dadurch gekennzeichnet, dass in dem 1,1-Dioxo-perhydro-1,2,4-thiadiazin der allgemeinen Formel (I) R¹ und R² jeweils für ein Wasserstoffatom (Taurultam) steht.

3. Verwendung nach Anspruch 1, dadurch gekennzeichnet, dass R¹ in der allgemeinen Formel (I) für ein Wasserstoffatom und R² für einen Rest der Formel (II) 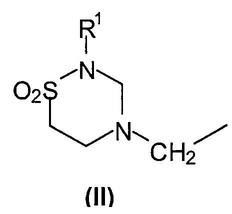 (Taurolidin) steht.

4. Verwendung nach einem der Ansprüche 1 bis 3, dadurch gekennzeichnet, dass die Konzentration an 1,1-Dioxoperhydro-1,2,4-thiadiazin einschließlich Metaboliten 0,005 bis 10,00 Gew.-%, jeweils bezogen auf das Gesamtgewicht der Zubereitungen, beträgt.

5. Verwendung nach einem der Ansprüche 1 bis 4, dadurch gekennzeichnet, dass die Konzentration an 1,1-Dioxoperhydro-1,2,4-thiadiazin einschließlich Metaboliten 0,01 - 5,0 Gew.-% , jeweils bezogen auf das Gesamtgewicht der Zubereitungen, beträgt.

6. Verwendung nach einem der Ansprüche 1 bis 5 in Kombination mit weiteren üblichen an sich bekannten gegen unreine Haut und Akne wirksamen Mitteln.

7. Verwendung nach Anspruch 6, dadurch gekennzeichnet, dass das weitere Mittel ein Keratolytikum ist.

8. Verwendung nach Anspruch 7, dadurch gekennzeichnet, dass das Keratolytikum ausgewählt ist aus Salicylsäure, Milchsäure, Glycolsäure, Citronensäure oder Äpfelsäure.
